(19) Europäisches Patentamt
European Patent Office
Office européen des brevets

(11) Publication number: 0 200 495 B1

# (12) EUROPEAN PATENT SPECIFICATION

(45) Date of publication of patent specification: 19.07.89

(51) Int. Cl.[4]: C 07 D307/60

(21) Application number: 86303138.1

(22) Date of filing: 25.04.86

## (54) Pineapple ketone l'alkoxyalkyl derivatives.

(30) Priority: 29.04.85 US 728540

(43) Date of publication of application: 05.11.86 Bulletin 86/45

(45) Publication of the grant of the patent: 19.07.89 Bulletin 89/29

(84) Designated contracting states: AT BE CH DE FR GB IT LI NL SE

(56) References cited:
GB–A– 1 476 711
CHEMICAL ABSTRACTS, vol. 96, no. 23, June 7, 1982, Columbus, Ohio, USA. PICKENHAGEN, WILHELM; VELLUZ, ALAIN; PASSERAT, JEAN PIERRE; OHLOFF, GUENTHER: "Estimation of 2,5-dimethyl-4-hydroxy-3(2H)-furanon (FURANEOL) in cultivated and wild strawberries, pineapples, and mangoes", page 536, column 1, abstract no. 198098m & J. Sci. Food Agric. 1981, 32(II), 1132-4
CHEMICAL ABSTRACTS, vol. 88, no. 7, February 13, 1978, Columbus, Ohio, USA. KALLIO, HEIKKI PAAVO TAPIO; TAPANI; HONKANEN, ERKKI JOHANI: "Giving food or drinks, especially liquors, an aroma of wild berries", page 419, column 1, abstract no. 4925r & Finn. 51,890
CHEMICAL ABSTRACTS, vol. 88, no. 19, May 8, 1978, Columbus, Ohio, USA. ITEN, PETER XAVER; HOFMANN, ALFRED ANDRE; EUGSTER, CONRAD HANS: "3,4-Dimethoxyfuran: properties, calculations and some substitution reactions", page 514, column 2, abstract no. 136387f & Helv. Chim. Acta 1978, 61(1), 430-3

(73) Proprietor: HERCULES INCORPORATED
Hercules Plaza
Wilmington Delaware 19894 (US)

(72) Inventor: Byrne, Brian
2211 Hilltop Road Building No. 2
Mahwah New Jersey 07430 (US)
Inventor: Lawton, Louise Marie Lafleur
35 Glen Drive
Goshen New York 10424 (US)

(74) Representative: De Minvielle-Devaux, Ian Benedict Peter et al
CARPMAELS & RANSFORD 43, Bloomsbury Square
London WC1A 2RA (GB)

EP 0 200 495 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACTS, vol. 99, no. 9, August 29, 1983, Columbus, Ohio, USA. HIRVI, TIMO: "Mass fragmentographic and sensory analyses in the evaluation of the aroma of some strawberry varieties", page 500, column 1, abstract no. 69137m & Lebensm.-Wiss. Technol. 1983, 16(3), 157-61

## Description

This invention relates to derivatives of pineapple ketone having a flavor similar to that of pineapple ketone and reduced reactivity.

Pineapple ketone is the common name for the chemical 2,5-dimethyl-4-hydroxy-3(2H)-furanone, and its derivatives in which the alkyl groups are substituted, such as for instance, 2-ethyl-5-methyl-4-hydroxy-3(2H)-furanone, sometimes called « homo-pineapple ketone », or its derivatives in which one of the alkyl groups is substituted by hydrogen, sometimes called a « desmethyl-pineapple ketone ».

Pineapple ketone is found in pineapples, strawberries, raspberries meats, and other foods. It has been found in cooked, roasted and fermented foods including coffee, roasted filbert, roasted almond and soy sauce. Pineapple ketone is known to be formed by the non-enzymatic browning process that occurs during roasting and baking.

Because of its cotton-candy, caramelized-sugar flavor, pineapple ketone is used extensively to compound synthetic flavors. However, it reacts readily with amines, aldehydes and oxygen.

Hirvi et al., (Lebensm.-Wiss. u.-Technol., 13, 324 (1980)) have indicated how sensitive pineapple ketone is to oxidation. At pH 4, it has a half-life of 120 days, while at pH 7, it has a half-life of 12 days. Oxidation in dry consumables during shelf life, for instance, reduces the pineapple ketone content and the effectiveness of the flavor to three months or less. In perfumes, the instability of pineapple ketone causes changes in the fragrance profile and discoloration.

GB-A-1,476,711 discloses 2,5-dimethyl-4-methoxy-3(2H)-furanone as an active ingredient of perfume compositions but does not mention any advantageous properties of that compound compared with pineapple ketone.

One common use of pineapple ketone is in chewing-gum. In this use, however, its water solubility causes it to « wash out » quickly in saliva during the chewing process, resulting in rapid loss of flavor.

One solution to the problem of flavor-loss in chewing-gum, disclosed in European Patent Application 85304674.6 (Publication No. 0,167,376A), uses carbonates derived from pineapple ketone. The pineapple ketone carbonates are formed by reacting pineapple ketone with an ethyl chloroformate or other alkyl chloroformates in triethylamine. The chemical reactivity of the resulting compounds is reduced compared with pineapple ketone, while the flavor of the derivatives remains very similar to the flavor of pineapple ketone, although it is less intense. The reduced chemical reactivity of these derivatives, particularly their increased resistance to oxidation, allows their use in applications where the shelf life of pineapple ketone is inadequate or it is too reactive, as in dry foods, tobacco, and perfumes.

Furthermore, the carbonate derivatives are not as water soluble as pineapple ketone, which allows these derivatives to be preferentially dissolved by a chewing-gum base, inhibiting the transfer to saliva of the pineapple ketone-like flavor of the derivatives while in the mouth, and prolonging the taste.

However, the pineapple ketone carbonate derivatives do not hydrolize readily to pineapple ketone in saliva and their less intense flavor is a disadvantage.

According to the invention, a derivative of pineapple ketone having a similar flavor but reduced reactivity is a 1′-alkoxyalkyl ether having the general formula,

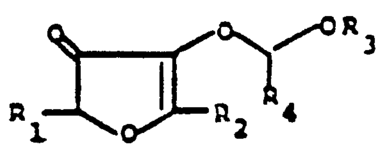

wherein $R_1$ and $R_2$ independently are $—H$, $—CH_3$, or $—CH_2CH_3$ ; $R_3$ is alkyl of from 1 to 10 carbons, alkenyl of from 2 to 10 carbons, or aryl of from 6 to 10 carbons ; and $R_4$ is hydrogen, alkyl of from 1 to 10 carbons, alkenyl of from 1 to 10 carbons or aryl of from 6 to 10 carbons.

The aryl radicals may optionally carry one or more substituents selected from hydroxy, alkoxy and alkyl groups.

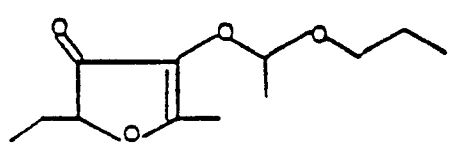

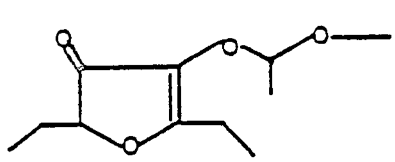

(Continued)

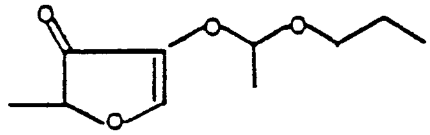

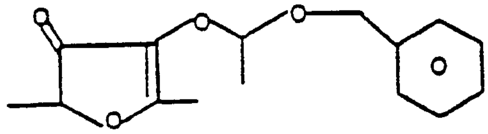

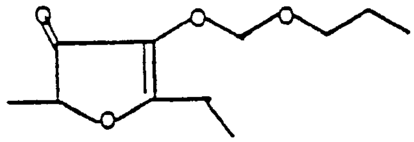

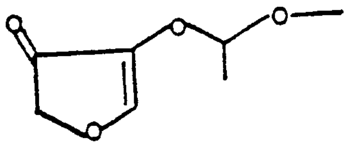

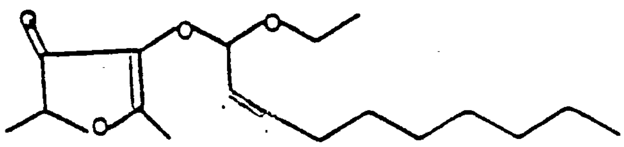

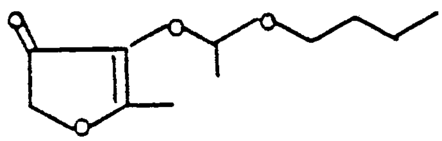

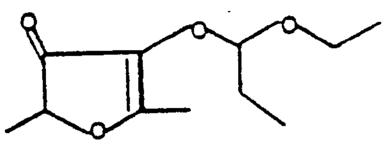

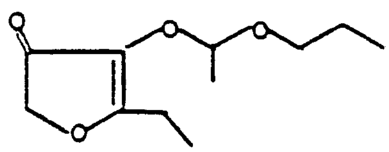

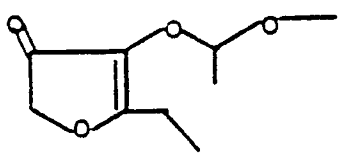

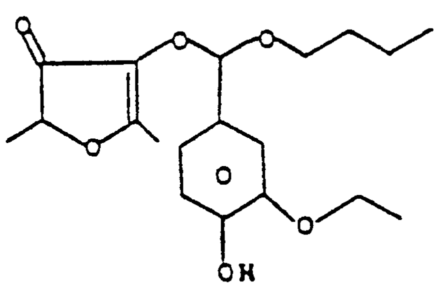

While the derivative of the invention has the advantages of the pineapple ketone carbonate derivatives, its slow-release from the chewing-gum base is accompanied by hydrolysis to the parent pineapple ketone in the slightly acid pH of saliva (about 6.2) thus producing the normally, intense flavor of the parent compound.

In particular, the pineapple ketone 1'-alkoxyalkyl ether according to the invention tastes like pineapple ketone, avoids « wash-out » by being preferentially soluble in chewing-gum base, and is less chemically reactive than pineapple ketone. It can be used in perfumes, and its resistance to oxidation in dry consumables preserves its flavor for a shelf life of up to 2 years.

The increased oxidative stability of the compounds according to the invention as compared to pineapple ketone is shown in Comparative Example 3.

Also according to the invention, a process for making a pineapple ketone 1'-alkoxyalkyl ether according to the invention comprises reacting pineapple ketone with ethyl vinyl ether or other 1-alkoxy-1-alkenes with an acid catalyst in trace amounts.

Examples of the preferred extended flavor compounds of the invention are :

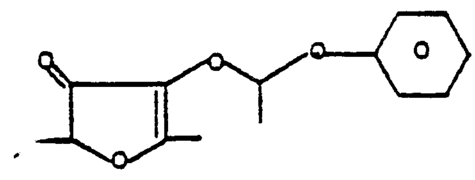

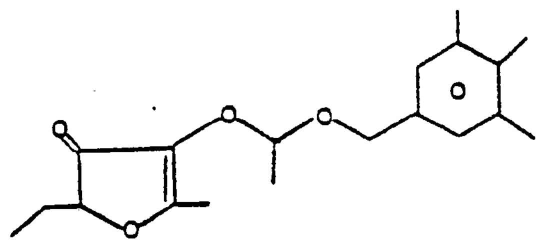

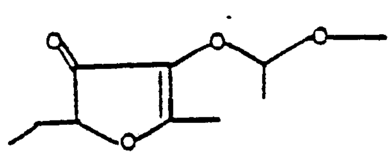

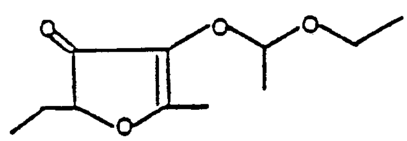

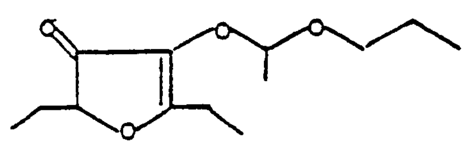

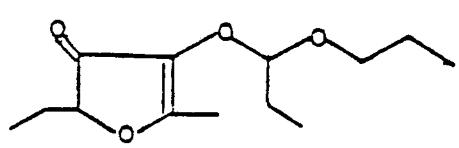

(Continued)

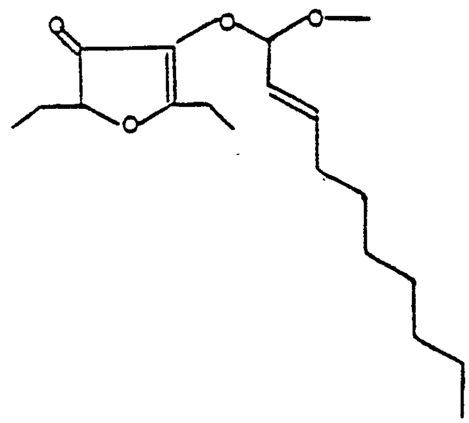

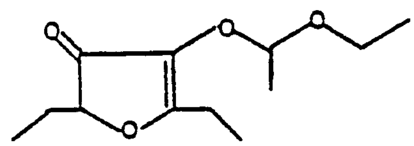

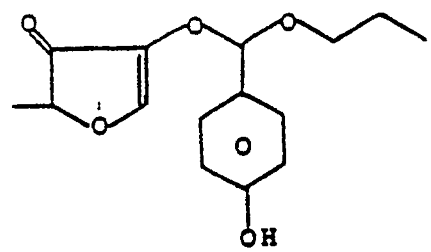

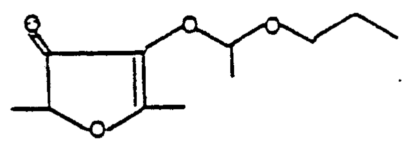

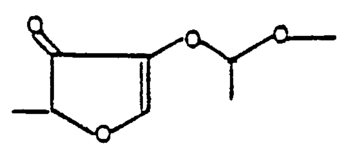

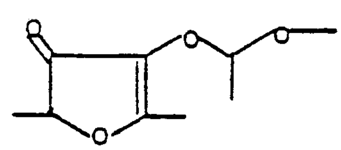

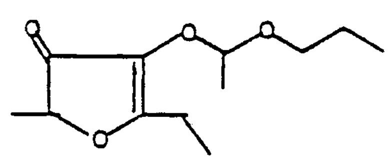

(Continued)

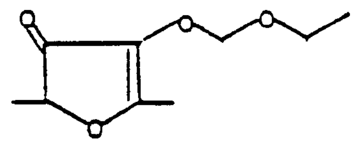

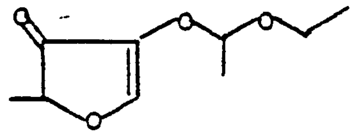

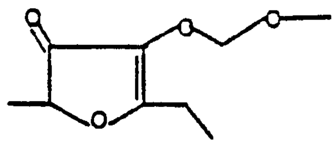

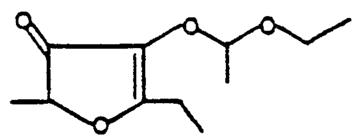

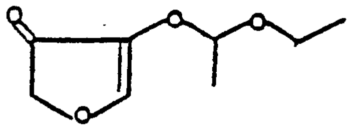

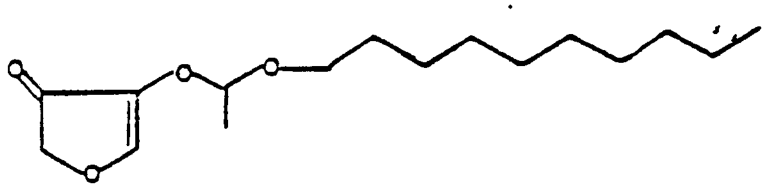

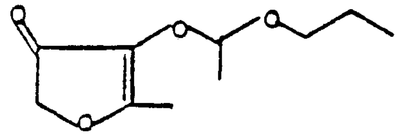

(Continued)

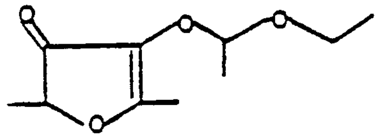

and

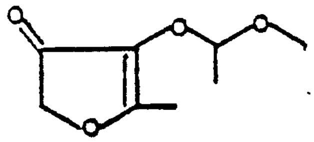

Example 1

Preparation of 2,5-Dimethyl-4-[(1′-ethoxy)ethoxy]-3(2H)-furanone

To a heterogeneous solution of 2,5-dimethyl-4-hydroxy-3(2H)-furanone (133.0 g, 1.0 mole) and ethyl vinyl ether (144.0 g 2.0 moles) at 10 °C, under $N_2$, is added dropwise, concentrated hydrochloric acid (1.0 g). The temperature rises to 11 °C and the solution becomes homogeneous. It is stirred for 1.5 hours, and then the coolant is removed. Stirring is continued at room temperature for an additional six hours, after which the reaction is quenched by the addition of solid sodium bicarbonate (10 g, 0.12 mole). The reaction mixture is washed with 100 ml water and a second time with 100 ml of 5 % $NaHCO_3$. The product is dried over $Na_2SO_4$ and distilled to give a 78.9 % yield (161.8 g) of 2,5-dimethyl-4-[(1′-ethoxy)ethoxy]-3(2H)-furanone, b. p. 91 °C (0.05 torr), IR (neat, film) 1 200, 1 625, 1 700, 2 930, 2 978 $cm^{-1}$ ; $^1H$ NMR ($CDCl_3$) 1.20 (t, J =8 Hz, 3), 1.32 (d, J = 5 Hz, 3), 1.42 (d, J = 7 Hz, 3), 2.2 (S, 3), 3.72 (m, 2), 4.48 (q, J = 7 Hz, 1), 5.32 (q, J = 5 Hz, 1).

Example 2

Preparation of 2,5-Dimethyl-4-[(1′-butoxy)ethoxy]-3(2H)-furanone

2,5-Dimethyl-4-hydroxy-3(2H)-furanone (13.3 g, 0.1 mole) and butyl vinyl ether (20.0 g, 0.2 mole) are reacted as in Example 1 to give a 37.8 % yield (8.83 g) of 2,5-dimethyl-4-[(1′-butoxy)ethoxy]-3(2H)-furanone, b. p. 68.8 °C (0.75 torr), IR (neat, film) 1 200, 1 625, 1 700, 2 865, 2 930, 2 958, 2 978 $cm^{-1}$, $^1H$ NMR ($CDCl_3$) 0.95 (m, 3), 1.35 (d, J = 5 Hz, 3), 1.38 (d, J = 8 Hz, 3), 2.18 (S, 3), 3.70 (m, 2), 4.48 (q, J = 7 Hz, 1) 5.31 (q, J = 5 Hz, 1).

Comparative Example 3

Oxidative Stability of Pineapple Ketone and the Product of Example 1

The comparative stability of pineapple ketone and the 1′-ethoxyethyl pineapple ketone derivative product of Example 1 are shown below. The disappearance of both compounds is measured by gas chromatography using a six foot long, one-eighth inch diameter column having 15 percent by weight polyethylene glycol with an average molecular weight of 20,000 (Carbowax-20M, Trademark) on particles of 80 by 100 mesh diatomaceous earth (Chromasorb W, Trademark) and using helium feed at 28 cc. per minute while heating from 100° to 210 °C at 8° per minute. Ten percent solutions of each compound in toluene are stirred at equal rates in the presence of air at ambient temperatures.

| Pineapple Ketone | | 2,5-Dimethyl-4[(1'-ethoxy)ethoxy]-3(2H)-furanone | |
|---|---|---|---|
| Time (Hours) | % Remaining | Time (Hours) | % Remaining |
| 0 | 100 | 0 | 100 |
| 15 | 24 | 17 | 95 |
| 41 | 18 | 41 | 93 |
| 115 | 6 | 116 | 44 |
| 138 | 4 | 137 | 38 |

Example 4

Bubble Gum

A typical bubble gum base is made with the following ingredients :

| | |
|---|---|
| Bubble Base T, Acid Balance (a gum base) (L. A. Dreyfus Co., South Plainfield, NJ) | 135.00 parts |
| Corn Syrup, 43° baume | 161.25 parts |
| Powdered sugar, confectioners 10X | 450.00 parts |
| Citric acid | 3.75 parts |
| Glycerine | 3.75 parts |

All of these ingredients are mixed in a gum blender with a jacketed sidewall. Gum A is formed by mixing 7.50 parts strawberry flavor 500389-U (Hercules, PFW Division, Middletown, NY) with the above parts of bubble gum base. Gum B is formed by mixing 7.30 parts strawberry flavor 500389-U, plus 0.20 parts of 2,5-dimethyl-4-[(1′-ethoxy)ethoxy]-3(2H)-furanone with parts of bubble gum base. The gums are cut into 5.0 gram pieces and evaluated by panelists. Both gums A and B have a long-lived strawberry flavor, with gum B having a higher overall rating for flavor prolongation after 10 minutes, as well as, having a higher and more sustained flavor intensity peak during the middle of the chew. Gum B is judged to have best retained the strawberry character throughout the chew.

**Claims** (for the Contracting States : BE, CH, DE, FR, GB, IT, LI, NL, SE)

1. A pineapple ketone derivative having the general formula,

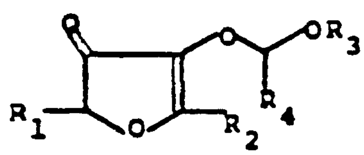

wherein $R_1$ and $R_2$ independently are —H, —$CH_3$, or —$CH_2CH_3$ ; and $R_3$ is alkyl of from 1 to 10 carbons, alkenyl of from 2 to 10 carbons, aryl of from 6 to 10 carbons or aryl of from 6 to 10 carbon atoms carrying at least one substituent selected from hydroxy, alkoxy and alkyl groups ; and $R_4$ is hydrogen, alkyl of from 1 to 10 carbons, alkenyl of from 1 to 10 carbons, aryl of from 6 to 10 carbons or aryl of from 6 to 10 carbon atoms carrying at least one substituent selected from hydroxy, alkoxy and alkyl groups.

2. A pineapple ketone derivative of the general formula given in claim 1, wherein $R_1$ and $R_2$ independently are —H, —$CH_3$, or —$CH_2CH_3$ ; and $R_3$ is alkyl of from 1 to 10 carbons, alkenyl of from 2 to 10 carbons, or aryl of from 6 to 10 carbons ; and $R_4$ is hydrogen, alkyl of from 1 to 10 carbons, alkenyl of from 1 to 10 carbons, or aryl of from 6 to 10 carbons.

3. The compound of claim 1 having the formulas :

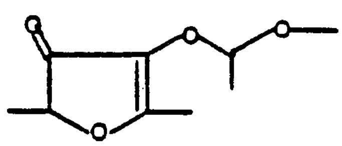

or

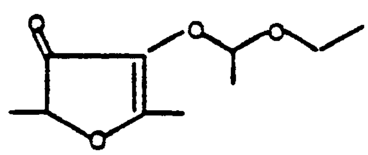

4. The compound of claim 1 having the formulas :

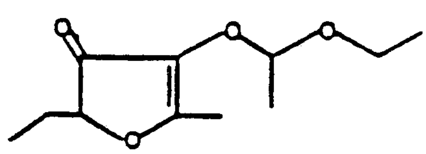

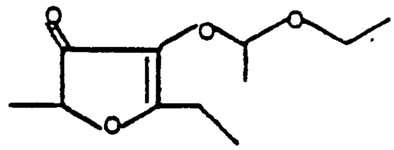

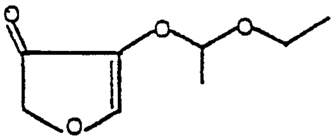

or

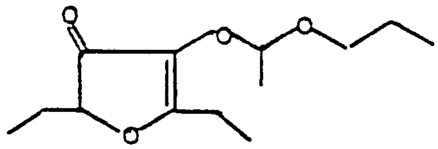

5. The compound of claim 1 having the formulas :

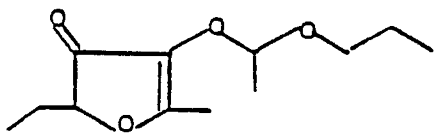

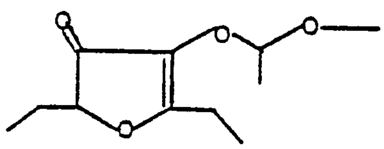

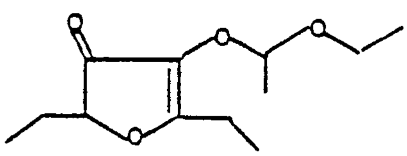

or

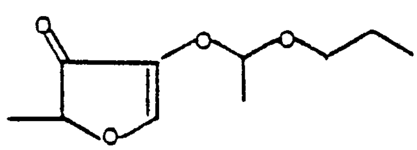

6. The compound of claim 1 having the formulas :

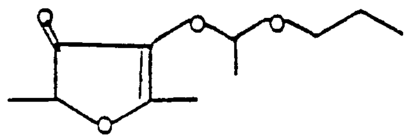

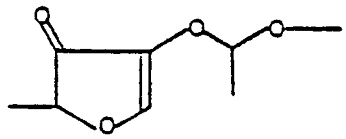

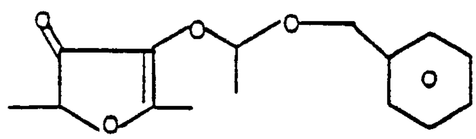

or

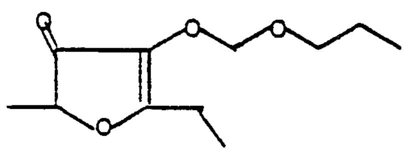

7. The compound of claim 1 having the formulas :

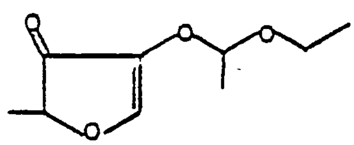

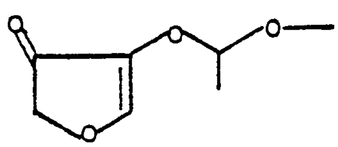

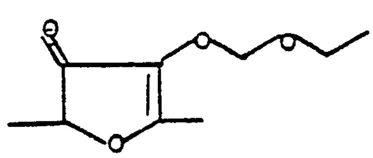

or

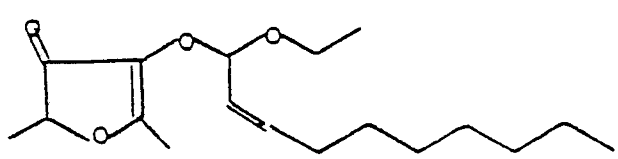

8. The compound of claim 1 having the formula :

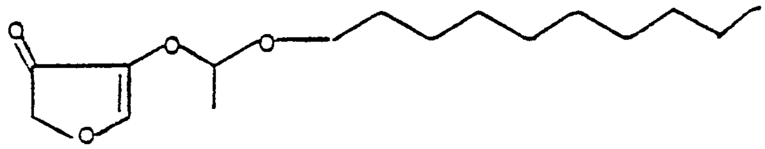

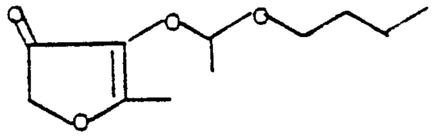

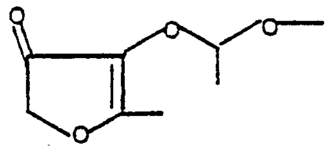

or

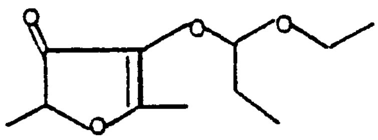

9. The compound of claim 1 having the formulas :

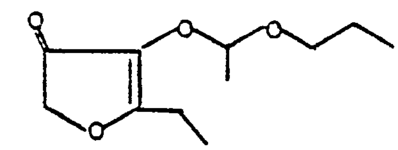

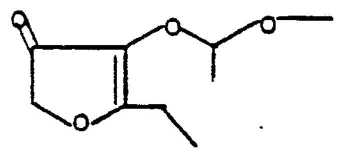

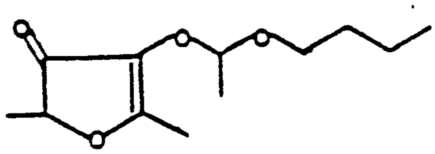

or

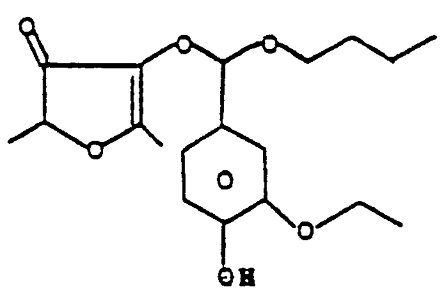

10. A process for making a pineapple ketone 1'-alkoxy-alkyl ether as claimed in any of claims 1 to 9, characterised in that it comprises reacting pineapple ketone with ethyl vinyl ether or other 1-alkoxy-1-alkene with a trace amount of an acid catalyst.

11. A chewing-gum containing a chewing-gum base having incorporated therein, as a flavouring agent, at least one compound according to any of claims 1 to 9.

**Claims** (for the Contracting State AT)

1. A process for making a pineapple ketone 1'-alkoxy-alkyl ether of the general formula,

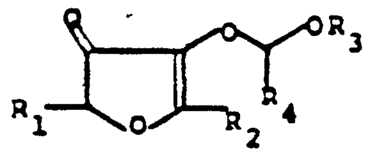

wherein $R_1$ and $R_2$ independently are —H, —$CH_3$, or —$CH_2CH_3$ ; and $R_3$ is alkyl of from 1 to 10 carbons, alkenyl of from 2 to 10 carbons, aryl of from 6 to 10 carbons or aryl of from 6 to 10 carbon atoms carrying at least one substituent selected from hydroxy, alkoxy and alkyl groups ; and $R_4$ is hydrogen, alkyl of from 1 to 10 carbons, alkenyl of from 1 to 10 carbons, aryl of from 6 to 10 carbons or aryl of from 6 to 10 carbon atoms carrying at least one substituent selected from hydroxy, alkoxy and alkyl groups, characterised in that it comprises reacting pineapple ketone with ethyl vinyl ether or other 1-alkoxy-1-alkene with a trace amount of an acid catalyst.

2. A process according to claim 1, characterised in that, in the general formula, $R_1$ and $R_2$ independently are —H, —$CH_3$, or —$CH_2CH_3$ ; and $R_3$ is alkyl of from 1 to 10 carbons, alkenyl of from 2 to 10 carbons, or aryl of from 6 to 10 carbons ; and $R_4$ is hydrogen, alkyl of from 1 to 10 carbons, alkenyl of from 1 to 10 carbons, or aryl of from 6 to 10 carbons.

3. A method of preparing a chewing-gum in which a flavouring agent is incorporated into a chewing-gum base, characterised in that the flavouring agent is a compound of the general formula as defined in claim 1 or 2.

**Patentansprüche** (für die Vertragsstaaten : BE, CH, DE, FR, GB, IT, LI, NL, SE)

1. Ein Ananasketonderivat der allgemeinen Formel

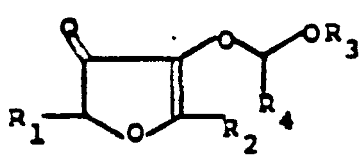

worin $R_1$ und $R_2$ voneinander unabhängig —H, —$CH_3$ oder —$CH_2CH_3$ sind ; und $R_3$ Alkyl mit 1 bis 10 Kohlenstoffatomen, Alkenyl mit 2 bis 10 Kohlenstoffatomen, Aryl mit 6 bis 10 Kohlenstoffatomen oder Aryl mit 6 bis 10 Kohlenstoffatomen, das wenigstens einen Substituenten, ausgewählt aus Hydroxy-, Alkoxy- und Alkylgruppen, aufweist, ist ; und $R_4$ Wasserstoff, Alkyl mit 1 bis 10 Kohlenstoffatomen, Alkenyl mit 1 bis 10 Kohlenstoffatomen, Aryl mit 6 bis 10 Kohlenstoffatomen oder Aryl mit 6 bis 10 Kohlenstoffatomen, das wenigstens einen Substituenten, ausgewählt aus Hydroxy-, Alkoxy- und Alkylgruppen, aufweist, ist.

2. Ein Ananasketonderivat der allgemeinen Formel nach Anspruch 1, worin $R_1$ und $R_2$ unabhängig voneinander —H, —$CH_3$ oder —$CH_2CH_3$ sind ; und $R_3$ Alkyl mit 1 bis 10 Kohlenstoffatomen, Alkenyl mit 2 bis 10 Kohlenstoffatomen, oder Aryl mit 6 bis 12 Kohlenstoffatomen ist ; und $R_4$ Wasserstoff, Alkyl mit 1 bis 10 Kohlenstoffatomen, Alkenyl mit 1 bis 10 Kohlenstoffatomen, oder Aryl mit 6 bis 10 Kohlenstoffatomen ist.

3. Verbindung nach Anspruch 1, welche die Formeln

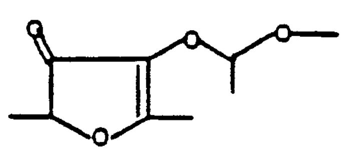

oder

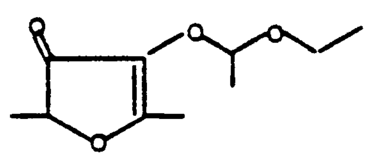

aufweist.

4. Verbindung nach Anspruch 1, welche die Formeln

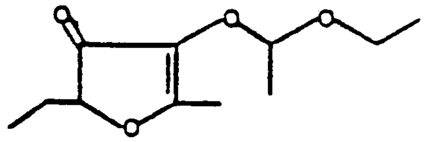

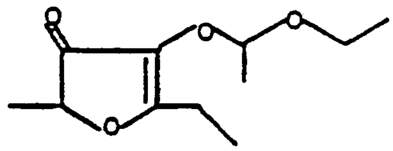

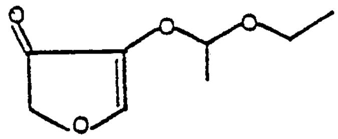

oder

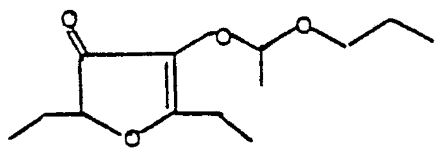

aufweist.

5. Verbindung nach Anspruch 1, welche die Formeln

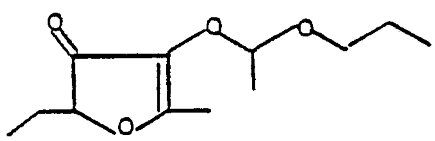

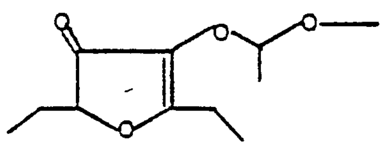

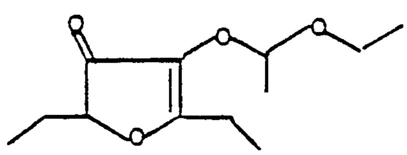

oder

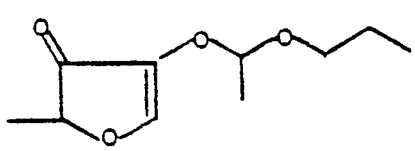

aufweist.

6. Verbindung nach Anspruch 1, welche die Formeln

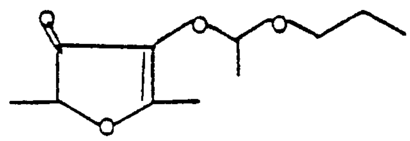

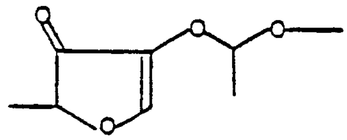

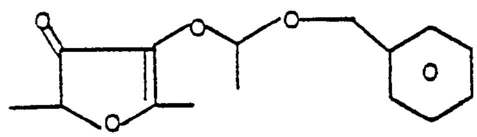

oder

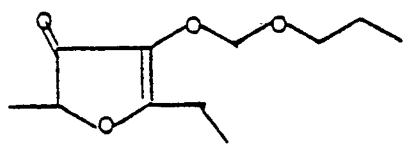

aufweist.

7. Verbindung nach Anspruch 1, welche die Formeln

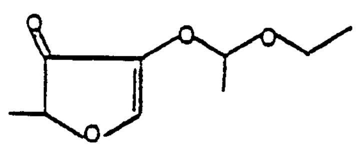

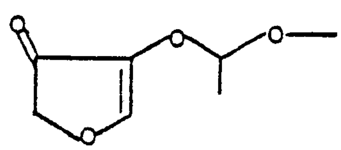

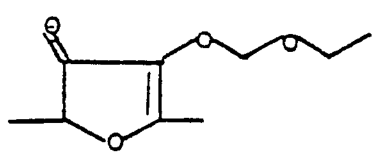

oder

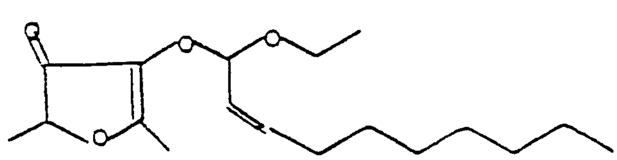

aufweist.

8. Verbindung nach Anspruch 1, welche die Formeln

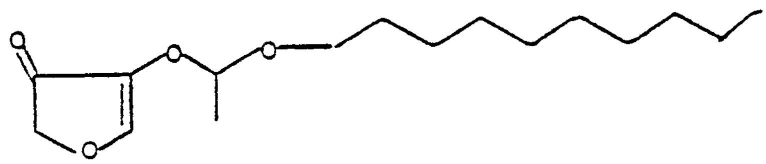

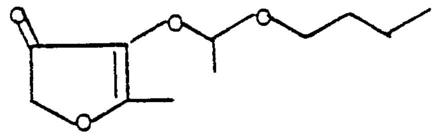

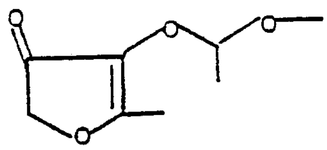

oder

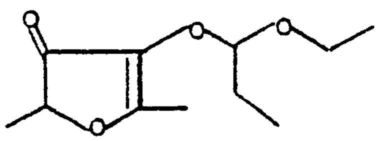

aufweist.

9. Verbindung nach Anspruch 1, welche die Formeln

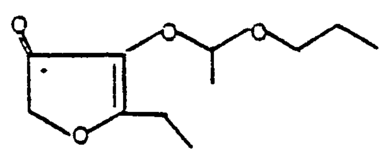

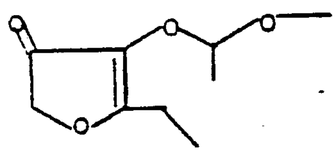

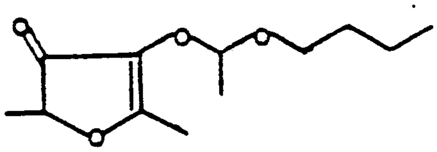

oder

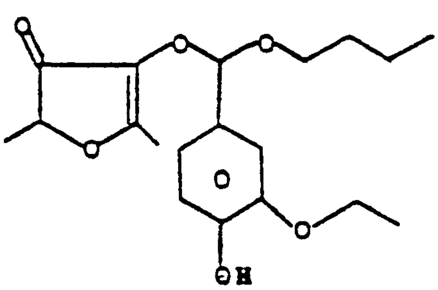

aufweist.

10. Verfahren zur Herstellung eines Ananasketon 1'-alkoxy-alkyl-ethers nach einem der Ansprüche 1 bis 9, dadurch gekennzeichnet, daß Ananasketon mit Ethylvinylether oder einem anderen 1-Alkoxy-1-Alken mit einer Spurenmenge eines Säurekatalysators zur Reaktion gebracht wird.

11. Kaugummi, der eine Kaugummibasis aufweist, in welche als Geschmacksstoff wenigstens eine Verbindung gemäß einem der Ansprüche 1 bis 9 eingearbeitet ist.

**Patentansprüche** (für den Vertragsstaat AT)

1. Verfahren zur Herstellung eines Ananasketon 1'-alkoxy-alkyl-Ethers der allgemeinen Formel

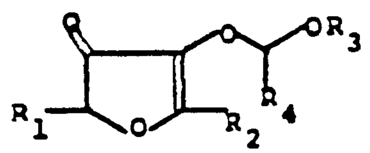

in welcher $R_1$ und $R_2$ voneinander unabhängig —H, —$CH_3$ oder —$CH_2CH_3$ sind; und $R_3$ Alkyl mit 1 bis 10 Kohlenstoffatomen, Alkenyl mit 2 bis 10 Kohlenstoffatomen, Aryl mit 6 bis 10 Kohlenstoffatomen oder Aryl mit 6 bis 10 Kohlenstoffatomen, das wenigstens einen Substituenten, ausgewählt aus Hydroxy-, Alkoxy- und Alkylgruppen, aufweist, ist; und $R_4$ Wasserstoff, Alkyl mit 1 bis 10 Kohlenstoffatomen, Alkenyl mit 1 bis 10 Kohlenstoffatomen, Aryl mit 6 bis 10 Kohlenstoffatomen oder Aryl mit 6 bis 10 Kohlenstoffatomen, das wenigstens einen Substituenten, ausgewählt aus Hydroxy-, Alkoxy- und Alkylgruppen, aufweist, ist, dadurch gekennzeichnet, daß Ananasketon mit Ethylvinylether oder einem anderen 1-Alkoxy-1-Alken mit einer Spurenmenge eines Säurekatalysators zur Reaktion gebracht wird.

2. Verfahren nach Anspruch 1, dadurch gekennzeichnet, daß in der allgemeinen Formel als $R_1$ und $R_2$ voneinander unabhängig —H, —$CH_3$ oder —$CH_2CH_3$ verwendet wird; und als $R_3$ Alkyl mit 1 bis 10 Kohlenstoffatomen, Alkenyl mit 2 bis 10 Kohlenstoffatomen oder Aryl mit 6 bis 10 Kohlenstoffatomen verwendet wird; und als $R_4$ Wasserstoff, Alkyl mit 1 bis 10 Kohlenstoffatomen, Alkenyl mit 1 bis 10 Kohlenstoffatomen oder Aryl mit 6 bis 10 Kohlenstoffatomen verwendet wird.

3. Verfahren zur Herstellung eines Kaugummis, in welchem ein Geschmacksstoff in die Kaugummibasis eingearbeitet ist, dadurch gekennzeichnet, daß als Geschmacksstoff eine Verbindung der allgemeinen Formel gemäß Anspruch 1 oder 2 eingesetzt wird.

**Revendications** (pour les Etats contractants : BE, CH, DE, FR, GB, IT, LI, NL, SE)

1. Dérivé de cétone d'ananas de formule générale

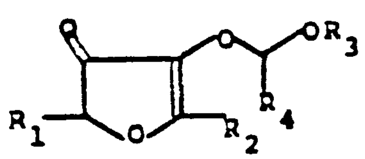

dans laquelle $R_1$ et $R_2$ représentent, indépendamment l'un de l'autre, —H, —$CH_3$ ou —$CH_2CH_3$ ; $R_3$ représente un reste alkyle à 1-10 atomes de carbone, alcényle à 2-10 atomes de carbone, aryle à 6-10 atomes de carbone ou aryle à 6-10 atomes de carbone portant au moins un substituant choisi entre des groupements hydroxy, alcoxy et alkyle ; et $R_4$ représente un atome d'hydrogène, un reste alkyle à 1-10 atomes de carbone, alcényle à 1-10 atomes de carbone, aryle à 6-10 atomes de carbone ou aryle à 6-10 atomes de carbone ou aryle à 6-10 atomes de carbone portant au moins un substituent choisi entre des groupements hydroxy, alcoxy et alkyle.

2. Dérivé de cétone d'ananas répondant à la formule donnée dans la revendication 1, dans laquelle $R_1$ et $R_2$ représentent, indépendamment l'un de l'autre, —H, —$CH_3$ ou —$CH_2CH_3$ ; $R_3$ représente un reste alkyle à 1-10 atomes de carbone, alcényle à 2-10 atomes de carbone ou aryle à 6-10 atomes de carbone ; et $R_4$ représente un atome d'hydrogène, un reste alkyle à 1-10 atomes de carbone, alcényle à 1-10 atomes de carbone ou aryle à 6-10 atomes de carbone ou aryle à 6-10 atomes de carbone.

3. Composé selon la revendication 1, répondant à la formule

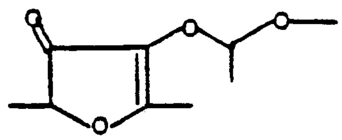

ou

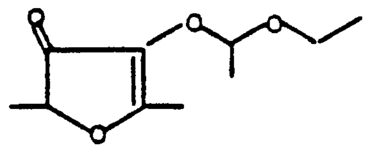

4. Composé selon la revendication 1, répondant à la formule

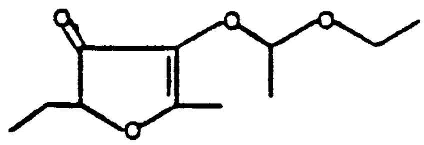

ou

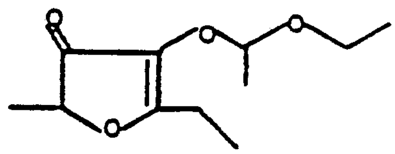

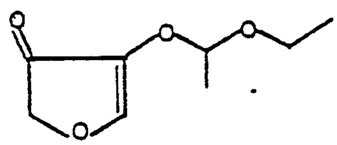

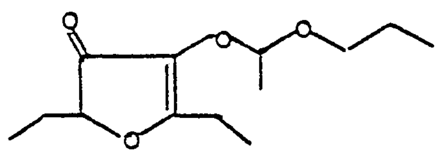

5. Composé selon la revendication 1, répondant à la formule

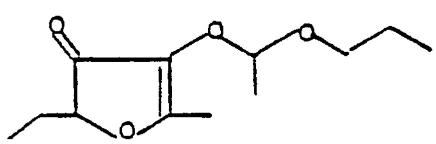

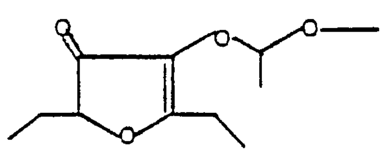

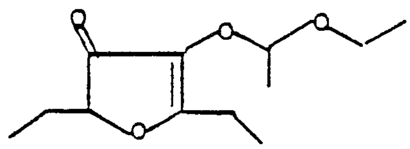

ou

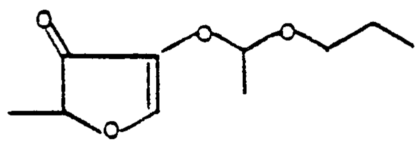

6. Composé selon la revendication 1, répondant à la formule

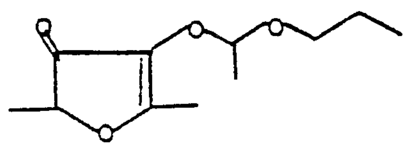

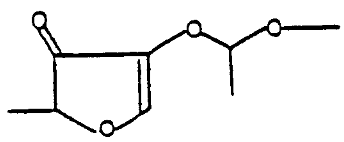

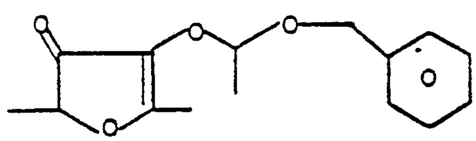

ou

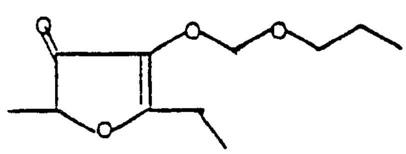

7. Composé selon la revendication 1, répondant à la formule

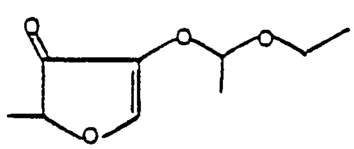

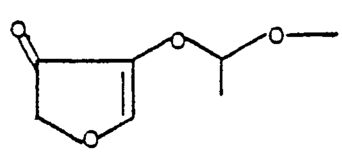

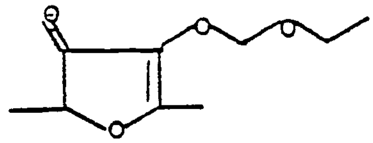

ou

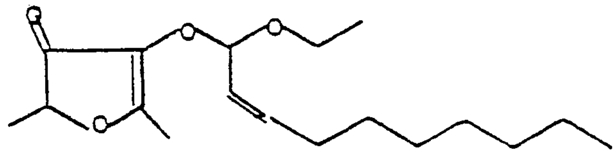

8. Composé selon la revendication 1, répondant à la formule

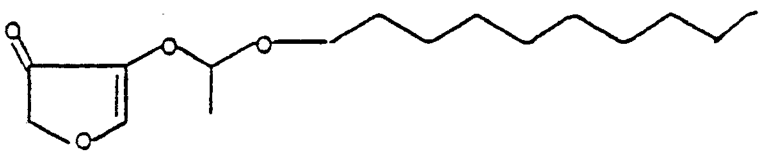

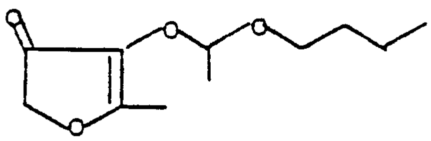

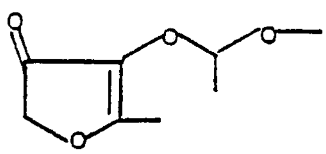

ou

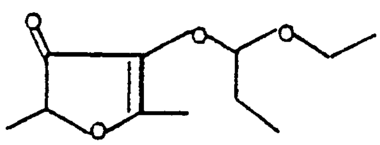

9. Composé selon la revendication 1, répondant à la formule

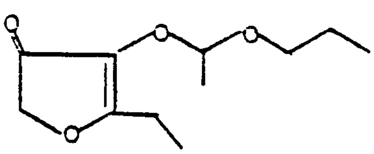

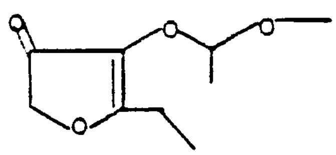

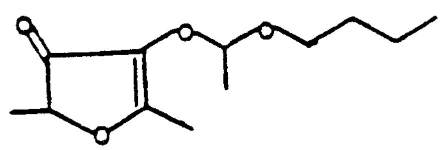

ou

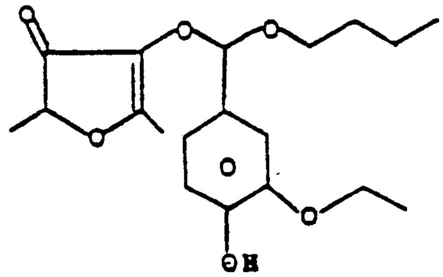

10. Procédé de préparation d'un 1′-alcoxyalkyléther de cétone d'ananas selon l'une quelconque des revendications 1 à 9, caractérisé en ce qu'il consiste à faire réagir une cétone d'ananas avec un éthylvinyléther ou un autre 1-alcoxy-1-alcène en présence d'une quantité de l'ordre d'une trace d'un catalyseur acide.

11. Chewing gum, contenant une base de chewing gum dans laquelle est incorporé, en tant que parfum, au moins un composé selon l'une quelconque des revendications 1 à 9.

**Revendications** (pour l'Etat contractant AT)

1. Procédé de préparation d'un 1′-alcoxyalkyléther de cétone d'ananas de formule générale

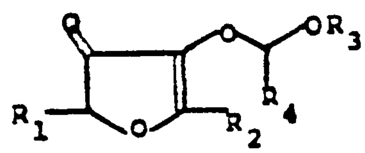

dans laquelle $R_1$ et $R_2$ représentent, indépendamment l'un de l'autre, —H, —$CH_3$ ou —$CH_2CH_3$ ; $R_3$ représente un reste alkyle à 1-10 atomes de carbone, alcényle à 2-10 atomes de carbone, aryle à 6-10 atomes de carbone ou aryle à 6-10 atomes de carbone portant au moins un substituant choisi entre des groupements hydroxy, alcoxy et alkyle ; et $R_4$ représente un atome d'hydrogène, un reste alkyle à 1-10 atomes de carbone, alcényle à 1-10 atomes de carbone, aryle à 6-10 atomes de carbone ou aryle à 6-10 atomes de carbone portant au moins un substituant choisi entre des groupements hydroxy, alcoxy et alkyle, caractérisé en ce qu'il consiste à faire réagir une cétone d'ananas avec un éthylvinyléther ou un autre 1-alcoxy-1-alcène en présence d'une quantité de l'ordre d'une trace d'un catalyseur acide.

2. Procédé selon la revendication 1, caractérisé en ce que dans la formule générale, $R_1$ et $R_2$ représentent, indépendamment l'un de l'autre, —H, —$CH_3$ ou —$CH_2CH_3$ ; $R_3$ représente un reste alkyle à 1-10 atomes de carbone, alcényle à 2-10 atomes de carbone ou aryle à 6-10 atomes de carbone ; et $R_4$ représente un atome d'hydrogène, un reste alkyle à 1-10 atomes de carbone, alcényle à 1-10 atomes de carbone ou aryle à 6-10 atomes de carbone.

3. Procédé de préparation d'un chewing gum, dans lequel un parfum est incorporé dans une base de chewing gum, caractérisé en ce que le parfum est un composé répondant à la formule générale, tel que défini dans la revendication 1 ou 2.